# EUROPEAN PATENT APPLICATION

(11) **EP 2 338 971 A1**
(43) Date of publication of application: **29.06.2011**
(21) Application number: 11000153.4
(22) Date of filing: 09.12.2005
(51) Int. Cl.: C12M 1/16

(54) **System and method for processing biomass**

(30) Priority: 10.12.2004 US 635235 P
(62) Divisional of application: 05853606.1
(71) Applicant: The Texas A&M University System, College Station, TX 77843-3369 (US)
(72) Inventor: Holtzapple, Mark, Texas 77840 (US); Davison, Richard, Texas 77801 (US); Granda, Cesar, Texas 77845 (US); Noyes, Gary, Texas 77062 (US); Darlington, Ed, Missouri 64012 (US)
(74) Representative: Dauncey, Mark Peter

(57) **Abstract**

A process and a method for treating biomass (105) comprising adding biomass into a vessel, adding an inoculum into said vessel, introducing liquid into the biomass from the bottom of the vessel, fermenting the biomass and removing biomass from the bottom of the vessel (102).

## Description

### RELATED APPLICATIONS

Pursuant to 35 U.S.C. § 119 (e), this application claims priority to United States Provisional Patent Application Serial No. 60/635,235, entitled SYSTEM AND METHOD FOR PROCESSING BIOMASS, filed December 10, 2004. United States Provisional Patent Application Serial No. 60/635,235 is hereby incorporated by reference.

### TECHNICAL FIELD OF THE INVENTION

The present invention relates generally to biomass processing and, more specifically, a system and method for the storage, pretreatment, and fermentation of biomass.

### BACKGROUND OF THE INVENTION

Processing biomass, especially waste biomass, to recover useful substances has been the focus of numerous efforts. Such treatments have used a variety of treatment methods and chemicals depending upon the desired recovery substance. Treatment with lime has been attempted, but usually at temperatures above 60°C for time frames of only a few weeks to a month. For example, previously issued patents to Holtzapple and Davison use high-temperature lime treatments to enhance enzymatic digestibility. One patent uses hot lime only and another uses hot lime plus high-pressure oxygen.

The most common methods for making pulp for paper or cardboard are Kraft and soda pulping. However, both of these methods use expensive chemicals and expensive treatment vessels. Additionally, previous methods and treatment systems often require movement of the biomass several times during the entire treatment process, including pretreatment and recovery.

### SUMMARY OF THE INVENTION

According to one embodiment of the invention, a system for processing biomass comprises a chamber, a biomass input device, a fluid input device, and a retrieval device. The chamber is defined by at least a bottom, at least one wall, and a cover supported by the at least one wall. The biomass input device operable to deliver biomass into the chamber to form a biomass pile. The fluid input device is operable to deliver fluid into the chamber to the biomass pile. The retrieval device operable to receive fluid from the chamber.

Certain embodiments of the invention may provide numerous technical advantages. For example, a technical advantage of one embodiment may include the capability to keep pretreators/fermentors at a low cost. Other technical advantages of other embodiments may include the capability to allow pretreatment and fermentation to occur in the same vessel. Yet other technical advantages of other embodiments may include the capability to remove spent solids.

Although specific advantages have been enumerated above, various embodiments may include all, some, or none of the enumerated advantages. Additionally, other technical advantages may become readily apparent to one of ordinary skill in the art after review of the following figures and description.

### BRIEF DESCRIPTION OF THE DRAWINGS

For a more complete understanding of example embodiments of the present invention and its advantages, reference is now made to the following description, taken in conjunction with the accompanying drawings, in which:
FIGURE 1 is a schematic of a system for processing biomass, according to an embodiment of the invention;
FIGURE 2A-2d illustrate a system for processing biomass, according to another embodiment of the invention;
FIGURE 3 shows a illustrate a system for processing biomass, according to another embodiment of the invention;
FIGURE 4 shows a cutaway view of a system for processing biomass, according to another embodiment of the invention;
FIGURE 5 shows a system for processing biomass, according to another embodiment of the invention;
FIGURE 6 shows a system for processing biomass, according to another embodiment of the invention;
FIGURE 7A and 7B shows rigid covers, according to embodiments of the invention;
FIGURE 8A, 8B, and 8C show a system for processing biomass, according to another embodiment of the invention;
FIGURE 9 shows a system for processing biomass, according to another embodiment of the invention;
FIGURE 10 shows a system for processing biomass, according to another embodiment of the invention;
FIGURE 11 shows a perspective view of a bottom of a chamber, according to an embodiment of the invention;
FIGURE 12 shows a perspective view of a bottom of a chamber, according to an embodiment of the invention;
FIGURE 13 shows multiple screw conveyors at a bottom of a chamber, according to an embodiment of the invention;
FIGURE 14 shows a system for processing biomass, according to another embodiment of the invention;
FIGURES 15 and 16 show screw conveyors in V-shaped sections, operable to move material towards a conveyor, according to an embodiment of the invention;
FIGURE 17 shows a system for processing biomass, according to an embodiment of the invention;
FIGURE 18 shows a system for processing biomass, according to another embodiment of the invention;
FIGURE 19 shows a system for processing biomass, according to another embodiment of the invention;
FIGURE 20 shows a cone, according to an embodiment of the invention;
FIGURE 21 shows a cone, according to an embodiment of the invention;
FIGURES 22 and 23 illustrates the use of a jet in conjunction with a cone, according to embodiments of the invention;
FIGURE 24 shows a cone formed in a floor, according to an embodiment of the invention;
FIGURE 25 shows a grate, according to embodiment of the invention;
FIGURE 26 shows an isometric view of a cut-away cone, according to embodiment of the invention; and
FIGURE 27, 28, and 29 show patterns for cones, according to embodiments of the invention.

### DETAILED DESCRIPTION OF EXAMPLE EMBODIMENTS OF THE INVENTION

It should be understood at the outset that although example embodiments of the present invention are illustrated below, the present invention may be implemented using any number of techniques, whether currently known or in existence. The present invention should in no way be limited to the example embodiments, drawings, and techniques illustrated below, including the embodiments and implementation illustrated and described herein. Additionally, the drawings are not necessarily drawn to scale.

As briefly identified in the Background, processing biomass, especially waste biomass, to recover useful substances has been the focus of numerous efforts. Accordingly, teachings of some embodiments recognize a system and method that converts biomass to carboxylic acids using a mixed culture of microorganisms. Further, teachings of some embodiments of the invention recognize an economical construction of biomass processing systems. Yet further, teachings of some embodiments of the invention recognize a system and methods for integrating pretreatment and fermentation into a single chamber or vessel. Additionally, teachings of some embodiments of the invention recognize a system and methods for removing spent solids from fermenting chambers.

Particular embodiment may be utilized to process biomass, for example, lignocellulosic biomass and other types of biomass, with lime or other alkali to yield useful recovery products. Other embodiments may be utilize other treatment methods. In addition, in some embodiments, the technology described herein may be utilized in conjunction with the technology described in U.S. Patent Application Serial Number 10/698,199, filed October 31, 2003 which is herein incorporated by reference.

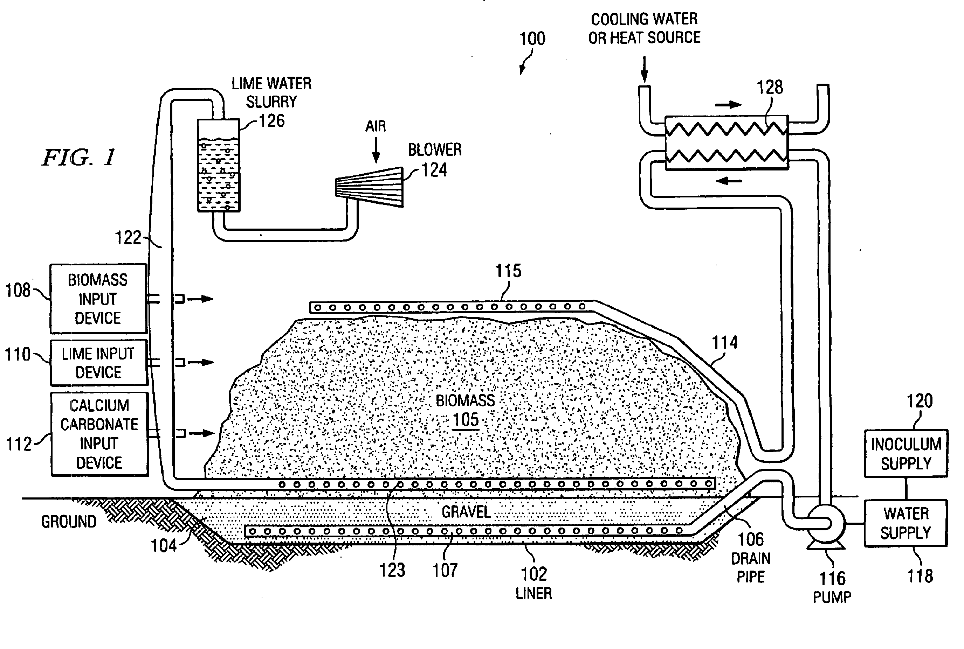

FIGURE 1 is a schematic of a system 100 for processing biomass 105, according to an embodiment of the invention. The system 100 of FIGURE 1 presents example components that may be utilized in such a processing of biomass. For purposes of brevity, the structural details of various components of system 100 are not shown or described. For example, the system may include a cover, which is not shown in FIGURE 1. Additionally, although specific components are shown with reference to the system 100 of FIGURE 1, other systems may utilize more, fewer, or different component parts.

In the embodiment of FIGURE 1, the system 100 includes a water-impermeable bottom liner 102, a gravel layer 104, a drainage device 106, a perforated pipe 107, a biomass input device 108, a lime input device 110, a calcium carbonate input device 112, a distribution device 114, a perforated pipe 115, a pump 116, a water supply 118, an inoculum supply 120, an air distribution device 122, a perforated conduit 123, an air blower 124, a lime water slurry container 126, and a heat exchanger 128. In particular embodiments, the system 100 may be utilized as a multi-use facility, which accepts and stores untreated biomass, pretreats the biomass, and ferments the biomass. Such a multi-use facility in particular embodiments may result in a reduction of biomass handling.

The liner 102 in particular embodiments may be formed of a water-impermeable material. In operation, the liner 102 supports the gravel layer 104 and prevents water or other materials from entering the ground. The liner 102 may be placed upon any suitable support. In the embodiment of FIGURE 1, the liner 102 is shown in a pit or bermed wall in the ground. The liner 102 may have any suitable shape and the depth. In particular embodiments, the liner 102 may be designed to handle a 'desired amount of gravel for the gravel layer 104. An example depth for gravel layer 104 is approximately three feet; however, other suitable depths may also be utilized for gravel layer 104. The gravel layer 104 may be comprised of any suitable loose or unconsolidated deposit of rounded pebbles, cobbles, boulders, or other suitable stone-like material that allow water to flow relatively freely therethrough.

Shown disposed on top of the gravel layer 104 in this embodiment is a pile of biomass 105 that may be delivered over gravel layer 104 via biomass input device 108. Biomass input device 108 represents any suitable device for creating biomass pile 105, such as a suitable conveyer system, front-end loader, or other suitable delivery system or device. As described above, the biomass in one embodiment, is lignocellulosic biomass, such as bagasse, corn stover, or other suitable biomass.

The lime input device 110 and the calcium carbonate input device 112 are any suitable devices operable to deliver lime and calcium carbonate, respectively, to the pile of biomass 105. In particular embodiments, the lime and/or calcium carbonate may be delivered while the pile of biomass 105 is formed so that the materials are evently distributed throughout. In other embodiments, lime and/or calcium carbonate may utilized to pretreat the biomass. Although the amount of lime added to the pile of biomass 105 may vary depending on the type of biomass 105, in one embodiment, an amount of lime delivered to the pile of biomass 105 is between approximately 10% and 30% of the biomass by weight.

Water from water supply 118 may be circulated through biomass pile 105 by pump 116 by delivering the water through distribution device 114, which may be any suitable device operable to distribute the water to the biomass pile 105. In particular embodiments, the distribution device 114 may include a perforated pipes 115 while in other embodiments the distribution device 114 may be spray head(s) or other suitable devices. After the water has traveled through the biomass pile 105 and gravel layer 104, the water is recovered through a drainage device 106, which may include a perforated pipe 107. In such an operation, circulation of the water may either be continuous with a relatively low flow rate or may be intermittent with a relatively high flow rate.

With a continuous circulation and low flow rate, channeling may occur which is undesirable because some portions of biomass pile 105 may not be wetted. Uneven wetting of biomass pile 105 may cause any one or more of the following problems: incomplete pretreatment of the pile of biomass 105, poor temperature control, and spontaneous combustion of dried portions of the pile of biomass 105. An intermittent circulation and high flow rate periodically floods the pile of biomass 105, thus ensuring all or most portions are wetted, thereby overcoming the potential problems of continuous circulation with low flow rate.

The temperature of the water circulated through the pile of biomass 105 may be regulated with the heat exchanger 128. The heat exchanger 128 may be any suitable device used to control the temperature of the water circulated through biomass pile 105. For example, heat exchanger 128 may be a shell-and-tube type heat exchanger designed to offload thermal energy.

While biomass pile 105 is being pretreated, air may be blown upward through biomass pile 105 to enhance lignin removal by alkaline oxidation. This may be facilitated by air blower 124 forcing air through air distribution device 122, which may include in particular embodiments a perforated conduit 123 disposed inside the gravel layer 104. Because air contains carbon dioxide, it may react with lime to form calcium carbonate, an unproductive reaction. To prevent this from occurring in biomass pile 105, the air may be scrubbed of carbon dioxide by passing it through lime water slurry in container 126, which may be a suitable packed column or tank. Oxygen enriched air flow may also be used.

The pile of biomass 105 may be subjected to a fermentation process while disposed over gravel layer 104. To facilitate the fermentation after pretreatment is complete, water may be circulated through biomass pile 105 that contains an inoculum of acid-forming microorganisms obtained from inoculum supply 120. The acid-forming microorganism start to degrade pile of biomass 105, forming carboxylic acids that react with calcium carbonate to form calcium carboxylate salts. Water may then be circulated through the pile of biomass 105 to remove the carboxylate salts.

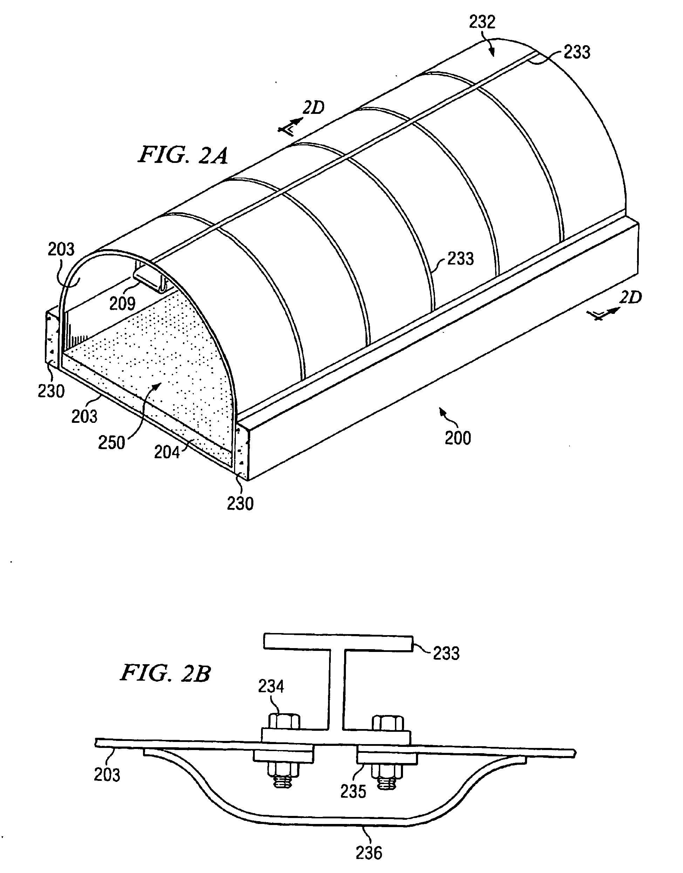

The storage, pretreatment, and fermentation of biomaas may also be accomplished using other suitable storage facilities or systems. Various embodiments of these systems are described below in conjunction with FIGURES 2A-2D. The components described with reference to FIGURE 1 may be utilized in conjunction with any of the systems described with reference to FIGURES 2A-2D.

FIGURE 2A-2D illustrate a system 200 for processing biomass. 205, according to another embodiment of the invention. FIGURE 2A shows an isometric view of a portion of the system 200. The system 200 is similar to the system 100 of FIGURE 1 except that system 200 includes a geomembrane 203, a cover 232, support ribs 233, walls 230, and a conveyor 209. As described in further details below, the various components of the system 200 may form a chamber 250 that can be used for storing untreated biomass, pretreating the biomass, and fermenting the biomass.

The geomembrane 203 may be formed from any suitable material and may perform a similar function to the liner 102 of FIGURE 1. In particular embodiments, the geomembrane 203 may line a substantial portion of the chamber 250. The geomembrane 203 in this embodiment is disposed beneath a.gravel layer 204 on a bottom portion of the system 200. The geomembrane 203 lines the interior of the walls 230 and extends over chamber 250 with support from the support ribs 233 to form the cover 232.

The walls 230 in this embodiment may be made of concrete. In other embodiments, the walls 230 may be made of other suitable materials. In particular embodiments, the walls 230 may extend above a ground level. In other embodiments, the walls may extend into the ground.

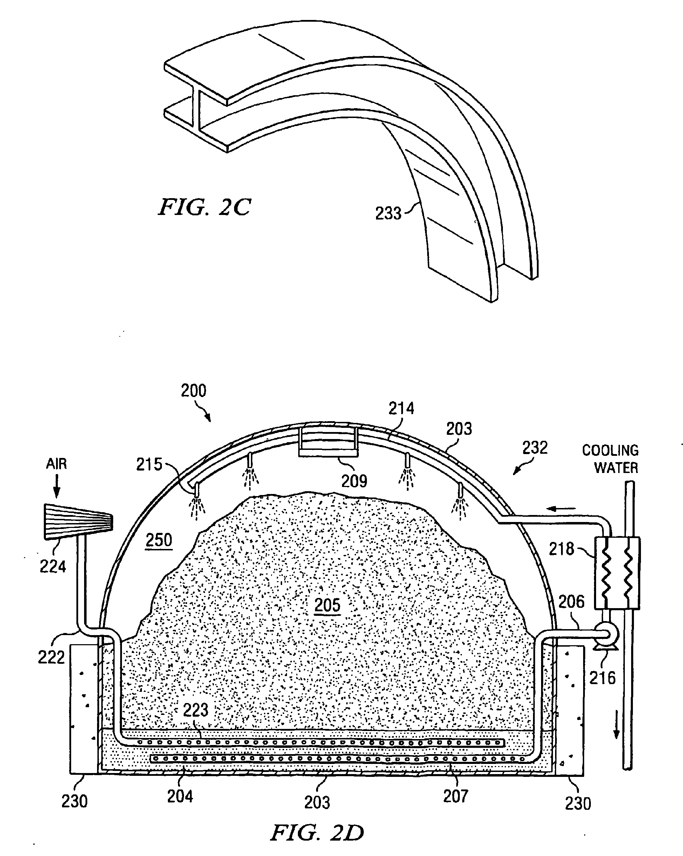

The support ribs 233 may be any suitable structure that can provide support for geomembrane 203 to help form the cover 232. For example, with reference to FIGURE 2C, the support ribs 233 are shown as an I-beam. However, the support ribs 233 may also be other suitable structural members such as a lightweight truss.

The geomembrane 203 may be coupled to the support ribs 233 in any suitable manner. FIGURE 2B illustrates one embodiment of coupling the support ribs 233 (shown as an I-Beam in this embodiment) to the geomembrane 203. With reference to FIGURE 2B, one or more bolts 234 are utilized to couple geomembrane 203 to the support ribs 233. Other suitable fasteners other than bolts may also be utilized to couple geomembrane 203 to the support ribs 233. A pair of stiffener plates 235 may provide stiffness to geomembrane 203, which is disposed between the stiffener plates 235 and the support ribs 233 and coupled therebetween by bolts 234. To prevent the corrosion of the bolts 234 and the stiffener plates 235, a boot 236 formed from the similar or different material than the geomembrane 203 may be utilized to cover the bolts 234 and stiffener plates 235.

The conveyor 209 may operate to dispose the biomass 205 (seen in FIGURE 2D) on top of the gravel layer 204 in the chamber 250. The conveyor 209 may be supported by the support ribs 233, running along the length of the system 200.

FIGURE 2D shows a cross-sectional view of the system 200 of FIGURE 2A, illustrating additional details of system 200 according to an embodiment of the invention. Biomass 205 is shown disposed on top of the gravel layer 204 on a bottom portion of the system 200. A perforated pipe 223 of an air distribution device 222 is embedded into the gravel layer 204 allowing compressed air from the air blower 224 to be blown up through the pile of biomass 205. Also, a perforated pipe 207 of the drainage device 206 is embedded into the gravel layer 204 to allow liquid to be pumped via pump 216 from the gravel layer 204 into sprayers 215 of distribution system 214 to wet the top of the pile of the biomass 205. In a manner similar to that described above with reference to FIGURE 1, during the pretreatment phase, air may be blown up through the pile of the biomass 205 while water is simultaneously trickled through the pile of the biomass 205. The action of air plus lime (which may be premixed into the biomass pile) removes lignin from the biomass, rendering it more digestible. When the lime is exhausted, the pH drops near neutrality. At this point, an inoculum of mixed acid-forming microorganisms may be added (e.g., using an innoculum supply 120 such as that shown in FIGURE 1), which digests the biomass 205 and converts it to mixed carboxylic acids. The acids react with calcium carbonate (which may be premixed into the biomass pile 205) to form carboxylate salts. The pump 216 circulates water through the pile of the biomass 205 to help extract the carboxylate salts as they are formed. During the above operations, the circulating liquid may go through a heat exchanger 228 to regulate temperature. Additionally, a portion of the circulating liquid may be passed to an adjacent chamber 250, which operates as a fermentor, that is operating at a higher carboxylate salt concentration. The circulating fluid may additionally be harvested and processed to recover the soluble product.

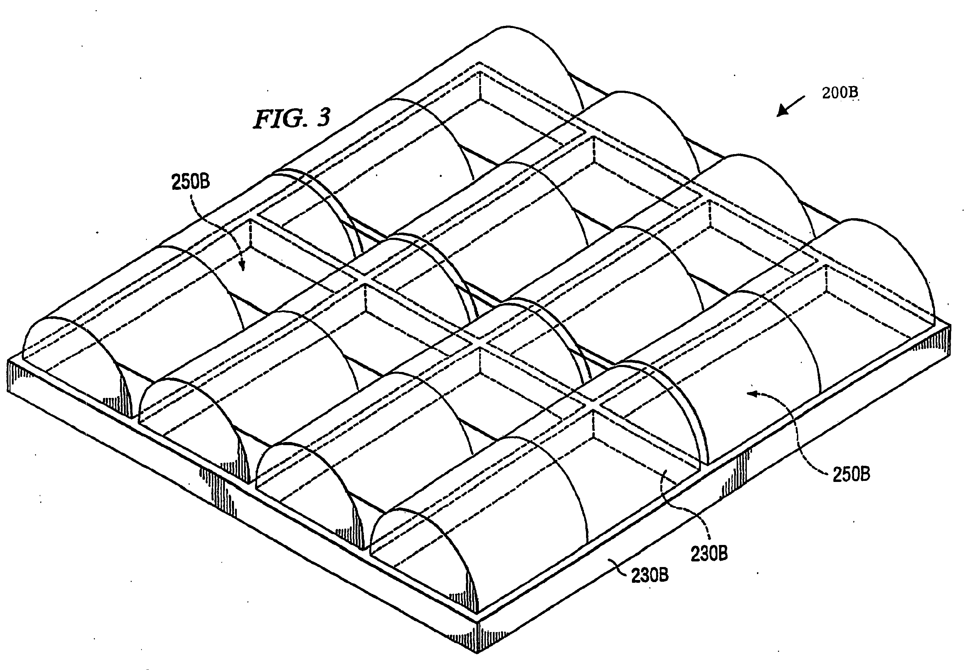

FIGURE 3 shows a illustrate a system 200B for processing biomass, according to another embodiment of the invention. The system 200B comprises eight-cells or chambers 250B. For purposes of brevity, the system 200B is shown in partial view with only particular components shown (e.g., walls 230B) and some components ghosted. However, each of the cells or chambers 250B may operate in a similar manner to the chambers 250 of system 200 described above. For example, each of the chambers 250B may operate to store untreated biomass, pretreat the biomass, and ferment the biomass. Additionally, each chamber 250B of system 200B of FIGURE 3 may include similar or different components than system 200. Further, each of the chambers 250B may operate as independent systems or systems that communicate with one another. The cells or chambers 250B may additionally be operated in a "round robin" manner. That is, one of the cells or chambers 250B would be in the process of filling with fresh biomass while another cell or chamber 250B would be in the process of removing spent solids. The other six cells or chambers 250B may be fermenting, each in a successive stage of digestion.

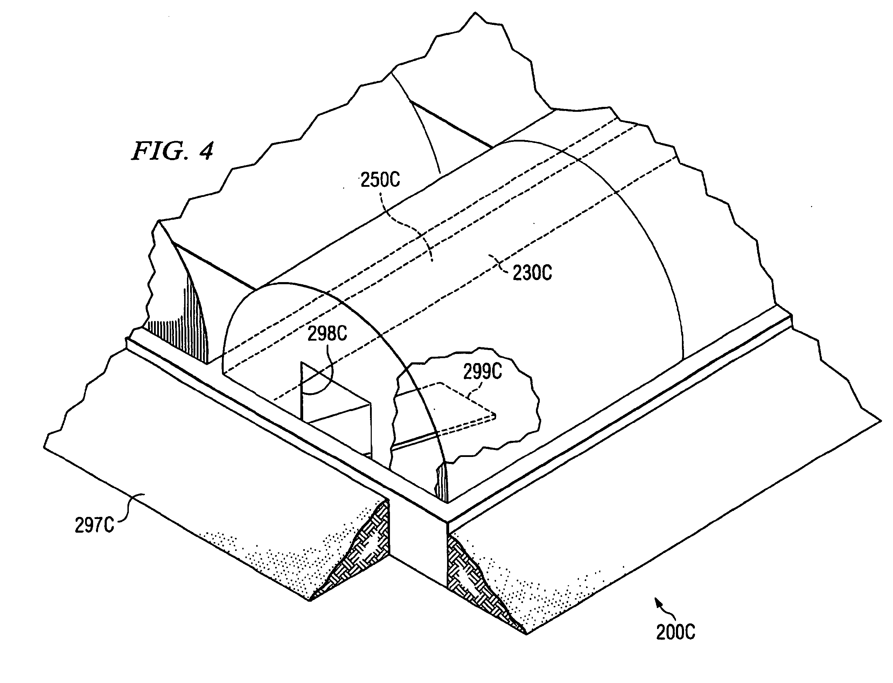

FIGURE 4 shows a cutaway view of a system 200C for processing biomass, according to another embodiment of the invention. The system 200C of FIGURE 4 shows how earth-moving equipment can enter a chamber 250C to remove spent solids in embodiments of the invention. In a manner similar to the system 200B of FIGURE 3, the system 200C of FIGURE 4 is in partial view with only particular components shown and some components ghosted. The system 200C in particular embodiments may include an elevated slop 297C, a wall 230C, a door 298C, and a slope passage 299C.

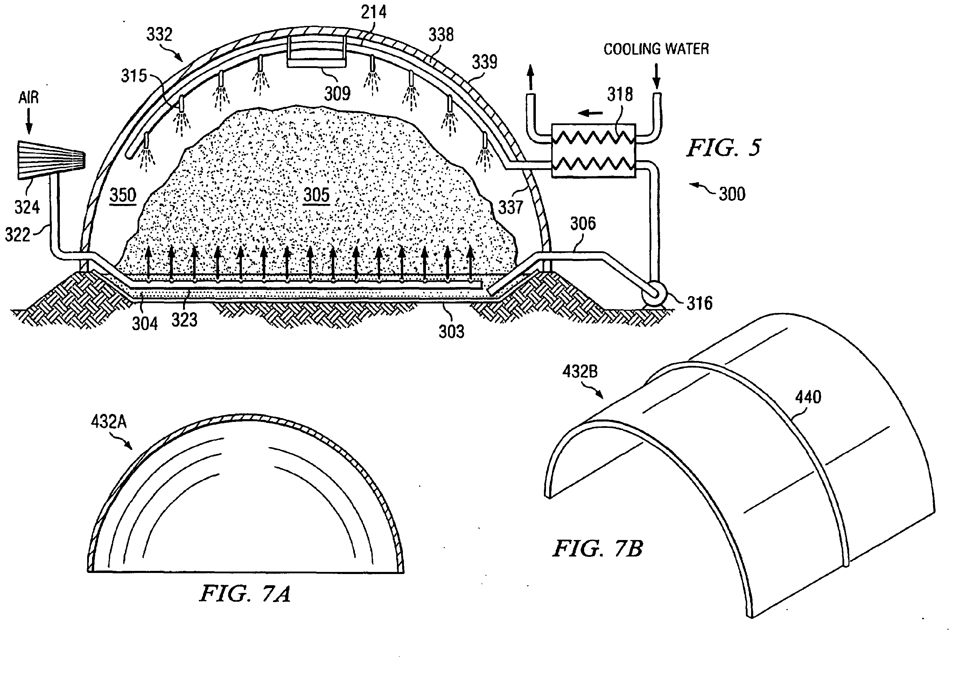

FIGURE 5 shows a system 300 for processing biomass 305, according to another embodiment of the invention. The system 300 is similar to system 200 of FIGURES 2A-2D, including a geomembrane 303, a gravel layer 304, a pile of biomass 305, a chamber 350, an air blower 324, an air distribution device 322 with a perforated pipe 323, a conveyor 309, a drainage device 306, a pump 316, a distribution system 314 with sprayers 315, a cover 332,
and a heat exchanger 328. The system 300 is different in that the system 300 does not include walls and the cover 332 is a rigid cover. The rigid cover 332 in this embodiment is shown with an inner core 338 between an interior layer 337 and an exterior layer 339. The inner core 338 may be made from a variety of materials, including, but not limited to "papercrete," a mixture of cement, sand, and paper pulp. The portions of each component of the papercrete can vary, but in particular embodiments includes the following mixture: cement 20%; sand 20%; and paper 60%. In other embodiments, the inner core 338 may be made of a papier mâché, a mixture of paper pulp and glue, or other suitable materials. The advantage of papercrete and papier mâché is that they can contain waste paper, which renders inner core 338 inexpensive. Also, the paper may act as an insulator, which helps regulate the temperature of the biomass pile. Because the paper component can be damaged when wetted, the inner layer 338 and the exterior layer 339 of the rigid cover 332 in some embodiments may be coated with a water-proofing material, such as tar.

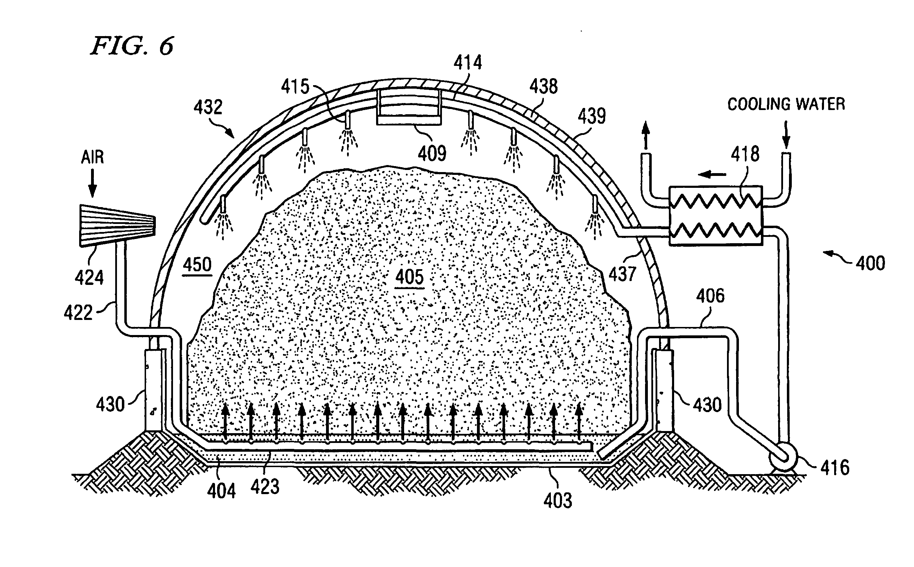

FIGURE 6 shows a system 400 for processing biomass 405, according to another embodiment of the invention. The system 400 is similar to system 300 of FIGURE 5, including a geomembrane 403, a gravel layer 404, a pile of biomass 405, a chamber 450, an air blower 424, an air distribution device 422 with a perforated pipe 423, a conveyor 409, a drainage device 406, a pump 416, a distribution system 414 with sprayers 415, a heat exchanger 428, and a rigid cover 432 having an inner core 438 sandwiched between an interior layer 437 and an exterior layer 439. The system 400 of FIGURE 6 additionally includes a concrete wall 430 surrounding a base of the system 400.

FIGURE 7A and 7B shows rigid covers 432A, 432B, according to embodiments of the invention. FIGURES 7A show a cut-away view of a domed rigid cover 432A that may be used with a circular chamber 450 and FIGURES 7B show a cut-away view of an arched rigid cover 432B that may be used with a rectangular chamber. Because the construction material is very light and could be blown over by the wind, the rigid covers 432A, 432B may be secured to the ground using a cable 440. The rigid covers 432A, 432B shown with reference to FIGURES 7A and 7B may be utilized with various embodiments of the invention.

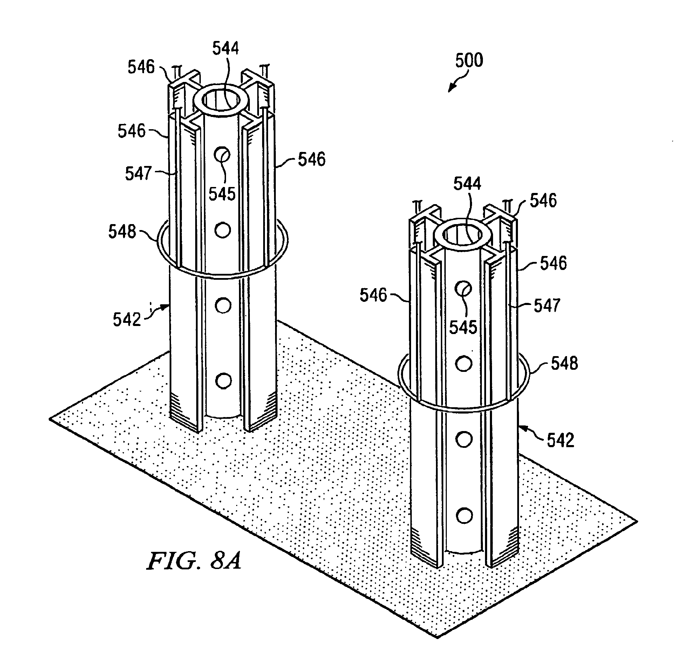

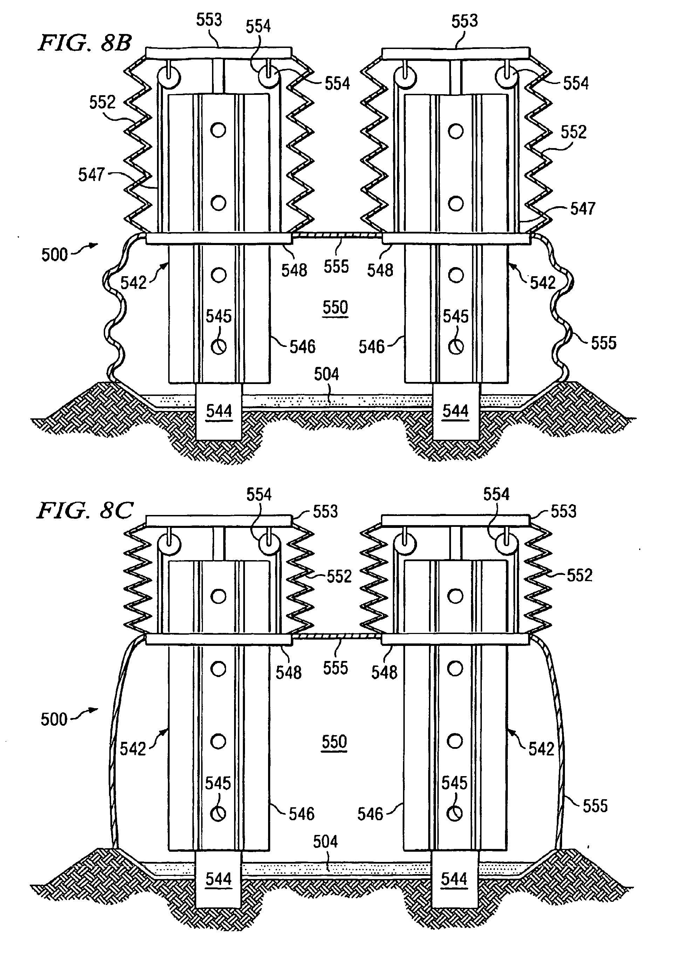

FIGURE 8A, 8B, and 8C show a system 500 for processing biomass, according to another embodiment of the invention. FIGURES 8A show tent poles 542 for the system 500. The tent poles 542 are shown protruding from a gravel layer 504. In this embodiment, the tent poles 542 include pipes 544 at the center with I-beams 546 welded to the exterior of the pipes 544. A hoop 548 surrounds the pole 544, which can be raised or lowered using a winch system (seen in FIGURE 8B), which can pull on cables 547. The pipes 544 may include perforations 545 at various levels. The perforations 545 allow a biomass pile to be constructed by pumping an aqueous slurry of the biomass through the pipes 544. In such an operation, the water carries the biomass to the pile and then drains away from the gravel base. The perforations 545 may also be used to circulate water through the pile during pretreatment or fermentation. Also, the perforations 545 may be used to deliver inoculum to the pile when the pretreatment is completed.

FIGURE 8B shows the tent poles 542 supporting a flexible cover 555, which is in the low position. The flexible cover 555 may be made of a variety of materials. A bellows 552, extending from a support 553 provides flexibility as the flexible cover 555 is raised and lowered. In particular embodiments, the bellows 552 may facilitate a substantial enclosure of the chamber 550. During fermentation, the flexible cover 555 can be lowered onto the biomass pile. A slight vacuum can be applied to the chamber 550 by sucking away fermentation gases. This ensures that the flexible cover 555 is sucked tightly against the pile, which prevents it from being damaged by the wind.

FIGURE 8C shows the flexible cover 555 in the raised position. The flexible cover 555 can be raised while the pile is being built, or when the solid residues are being removed. In such a raising of the flexible cover 555, the winch 554 can pull in the cables 547, which are attached to the rings 548, which are attached to the flexible cover 555.

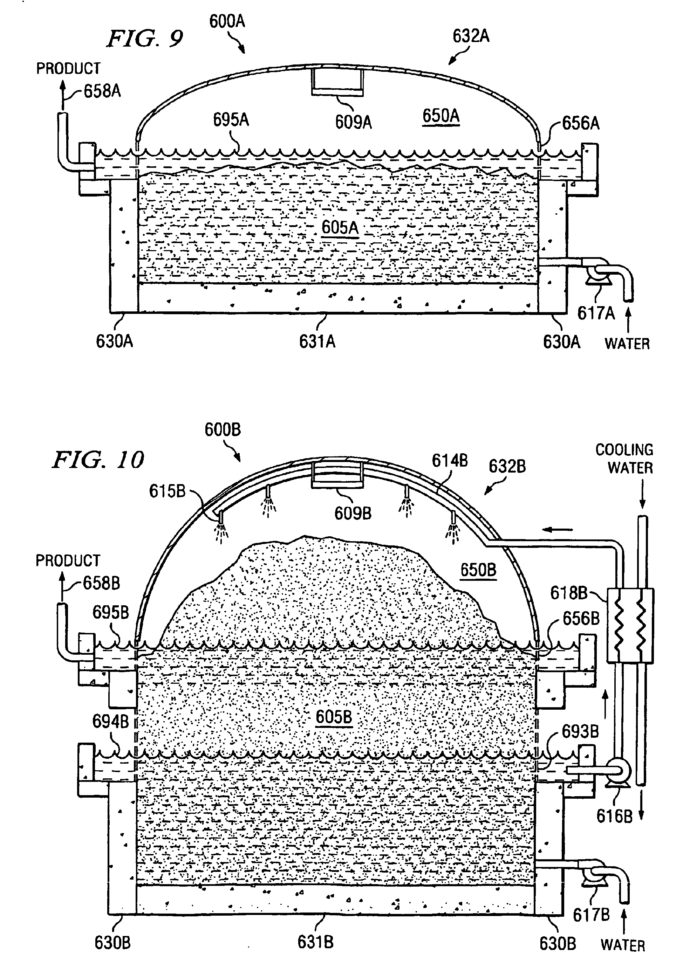

FIGURE 9 shows a system 600A for processing biomass 605A, according to another embodiment of the invention. The system 600A may be similar to system 400 of FIGURE 6. However, in the system 600A of FIGURE 9, fresh water is added via a pump 617A at the bottom through a distributor system so that it percolates up through the biomass 605A, which is submerged under a water line 695A. Fresh biomass 605A may constantly be added at the top of a chamber 650A, for example, through a conveyor 609A or other suitable mechanism and spent biomass may be removed from the bottom, using embodiments described herein or any suitable mechanism. Product with carboxylate salts may be removed from the top at the area indicated by arrow 658A, using any suitable device. In particular embodiments, a screen 656A may be employed to keep solids from entering the liquid product stream. A cover 632A for the system 600A may be any suitable cover, including rigid covers, flexible covers, and others. Walls 630A and a floor 631A may be made from any suitable material, including but not limited to concrete.

FIGURE 10 shows a system 600B for processing biomass 605B, according to another embodiment of the invention. The system 600B is similar to the system 600A of FIGURE 9 . except that the system 600B has a significant portion of the biomass 605B located above a first water line 695B. Liquid may be removed from a second water line 694B just below a first water line 695B, heat exchanged with a heat exchanger 628B to regulate temperature, and pumped via a pump 616B onto the sprayers 615B located in a distribution system 614B of an arched or domed cover 632B. The spray percolates through the biomass 605B above the first water line 695B. Product with carboxylate salts may be removed from the top at the area indicated by arrow 658B, using any suitable device. Screens 656B, 693B may allow removal of liquid while preventing passage of solids. The cover 632B for the system 600B may be any suitable cover, including rigid covers, flexible covers, and others. Walls 630B and a floor 631B may be made from any suitable material, including but not limited to concrete.

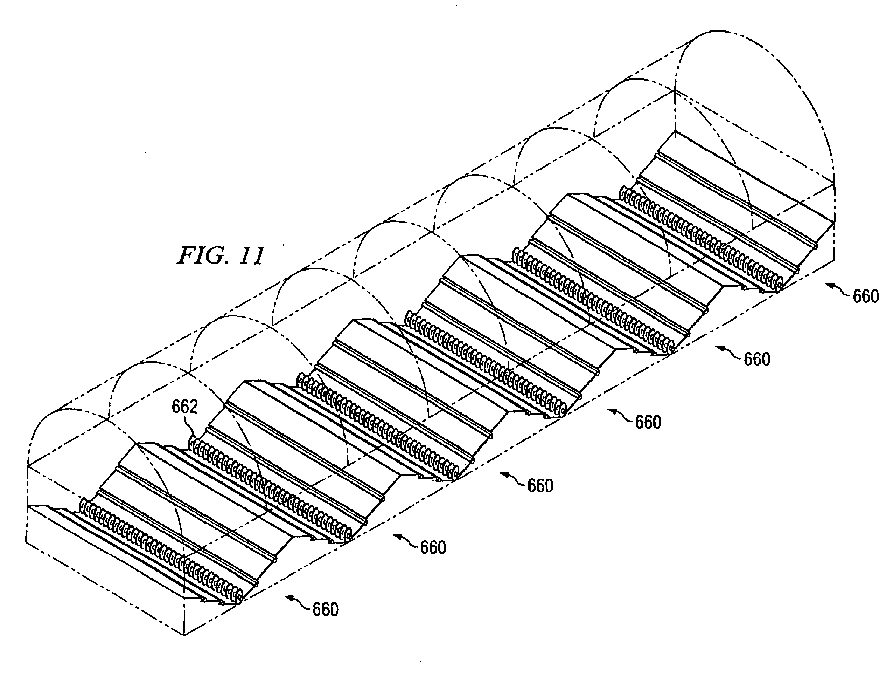

FIGURE 11 shows a perspective view of a bottom of a chamber 650, according to an embodiment of the invention. The bottom of the chamber 650, for example, may be the bottom of chambers 650A, 650B of FIGURES 9 and 10. The bottom of the chamber 650 , which as referenced above may be used as a fermentor, is shown as a series of V-shaped sections 660 with a screw conveyor 662 located at the tip of the V-shaped sections. In one embodiment, the slope of the "V" is greater than the angle of repose, allowing solids to flow toward the top of the V.

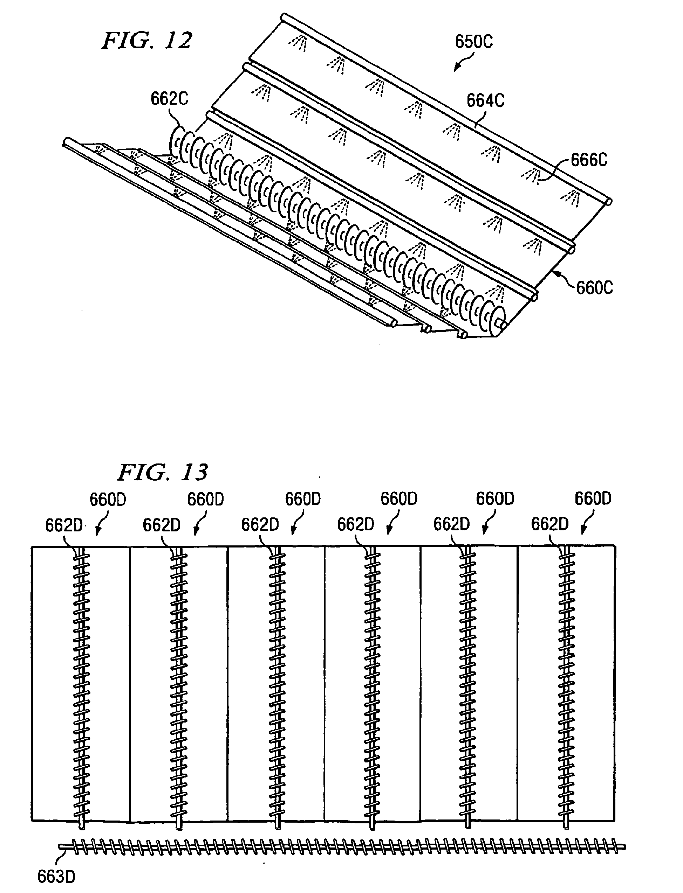

FIGURE 12 shows a perspective view of a bottom of a chamber 650C, according to an embodiment of the invention. To help lubricate the surface and force solids downward towards the tip of a V-shaped section 660C, the section 660C may have water distributors 664C that employ nozzles 666C that blast liquid downward, forcing the solids downward.

FIGURE 13 shows multiple screw conveyors 662D at a bottom of a chamber 650D, according to an embodiment of the invention. The screw conveyors 662 of the embodiment of FIGURE 13 convey spent solids to a side, where another conveyor 663D removes them for ultimate disposal.

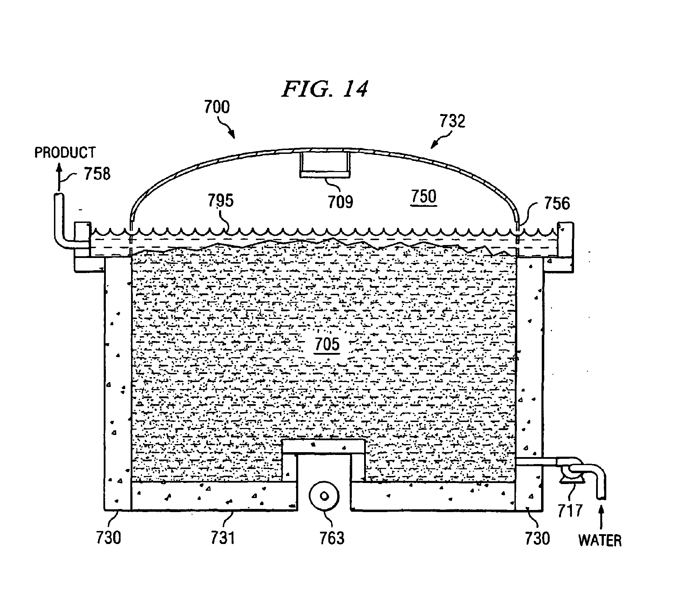

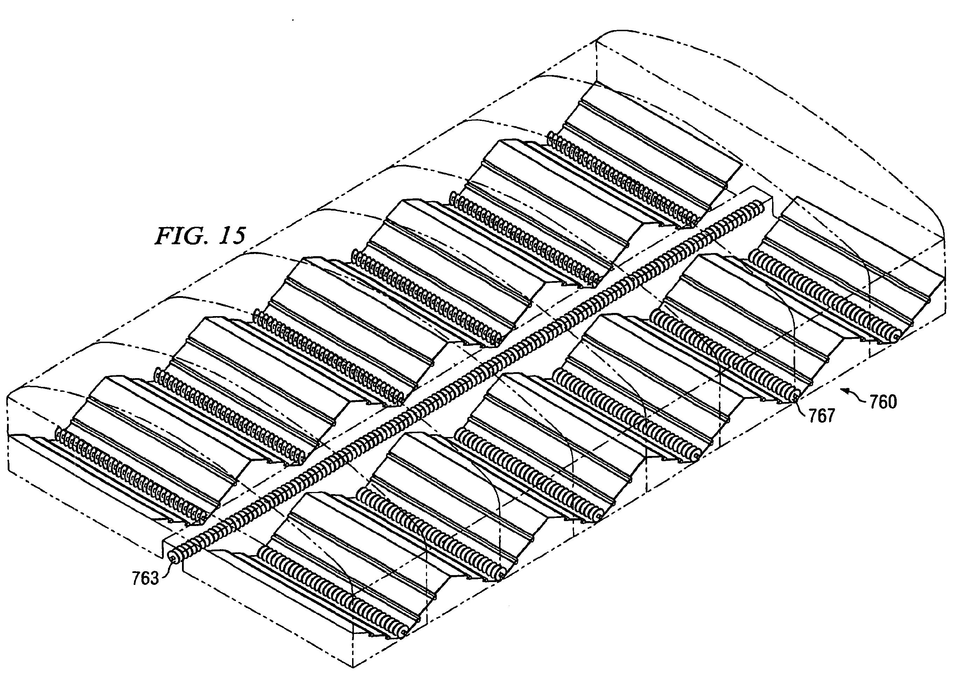

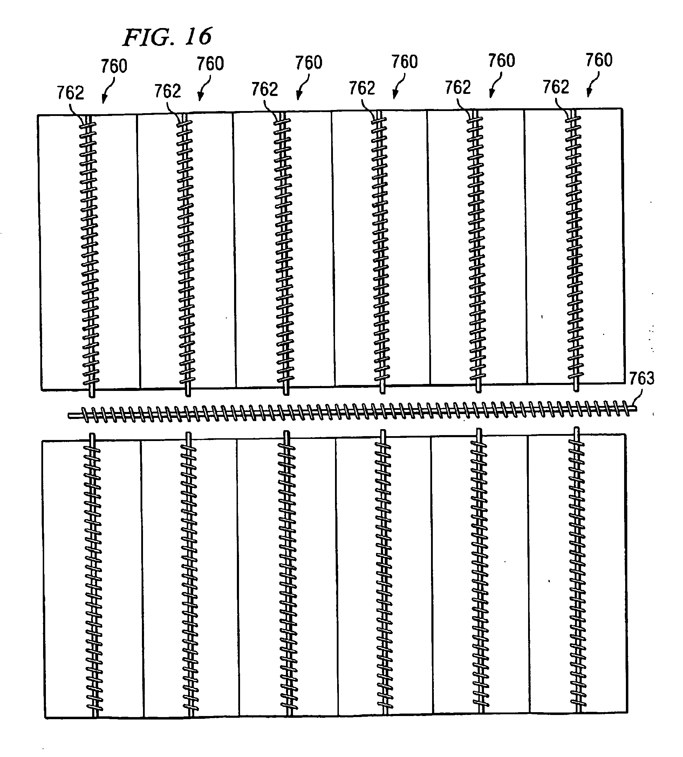

FIGURE 14 shows a system 700 for processing biomass, according to another embodiment of the invention. The system 700 of FIGURE 14 is similar to the system 600A of FIGURE 9, including walls 730B, a floor 731B, a water line 795B, a pump 717, a screen 756, biomass 750, product removal indicated by arrow 758, a cover 732, and a water line 795. However, system 700 includes a conveyor 763 that removes solids for ultimate disposal is located at the center of the chamber 750 rather than the side. The conveyor 763 is serviced by screw conveyors 760 on both sides, which is readily seen in FIGURES 15 and 16.

FIGURES 15 and 16 show screw conveyors in V-shaped sections 760, operable to move material towards a conveyor 763, according to an embodiment of the invention.

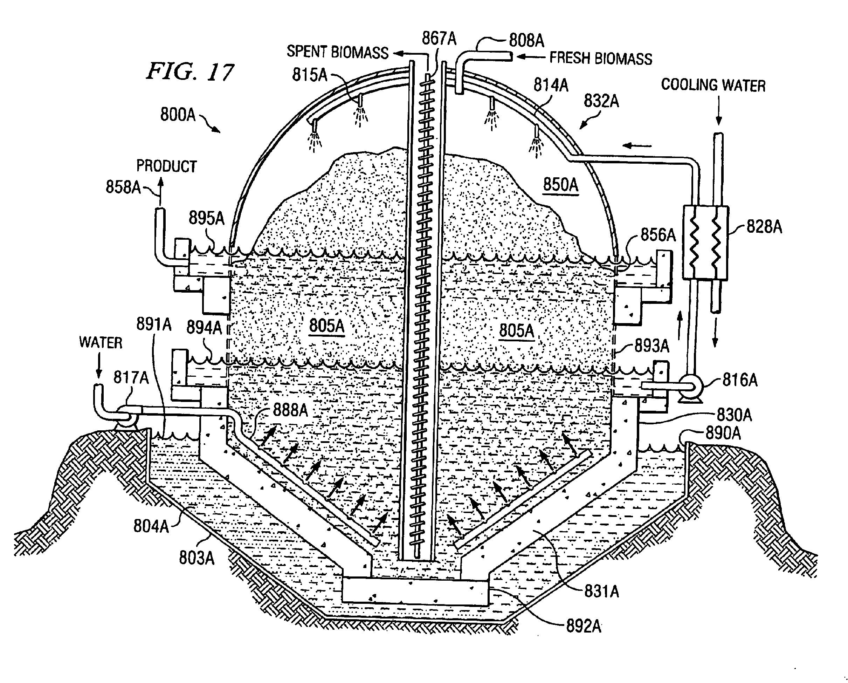

FIGURE 17 shows a system 800A for processing biomass 805A, according to an embodiment of the invention. The system 800A of FIGURE 17 is similar to the system 600B of FIGURE 10, including components such as walls 830A, a floor 831A, a first water line 895A, a second water line 894A, a heat exchanger 828A, a pump 816A, a pump 817A, a first screen 856A, a second screen 893A, sprayers 815A located in a distribution system 814A, product removal indicated by arrow 858, and a cover 832A. However, the system 800A has a single large cone 892A at the bottom of the chamber 850A that collects all spent solids. Further, the pump 817A pumps into a distribution device 888A. The biomass 805A may be introduced into the system using any of a variety of biomass input devices 808A. And, a conveyor 867A removes spent solids from the tip of the cone 892A and conveys them out the top of the cover 832A To provide a back-up barrier that would prevent leakage of contents of the chamber 850A into the groundwater should a leak develop, the floor 831A and/or wall 830A that help define the chamber 850A may be located in a hole 890A that is filled with a gravel layer 804A and lined with a geomembrane 803A. The space between the floor 831A and/or wall 830A that help define the chamber 850A and the geomembrane 803 may be filled with water 891A, which provides hydraulic head and helps balance the pressure of the liquid inside the chamber 850A. Such a configuration may allows thinner concrete structures, for example, in the floor 831A and/or walls 830A. Additionally, such a configuration may eliminate the need for prestressing the concrete with steel cables that keep the concrete in compression, where it is strongest.

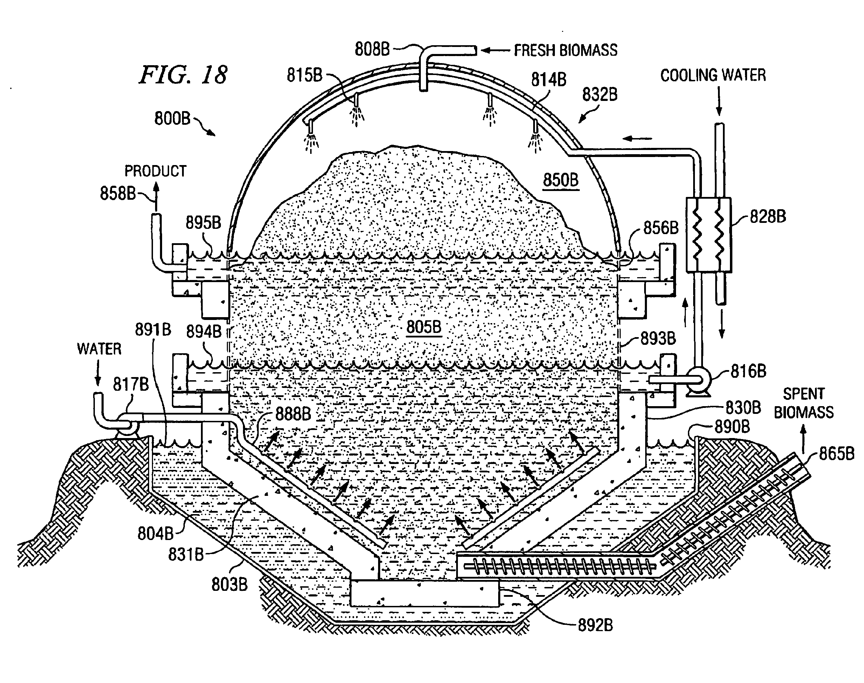

FIGURE 18 shows a system 800B for processing biomass 805B, according to another embodiment of the invention. The system 800B of FIGURE 18 is similar to the system 800A of FIGURE 17, including components such as walls 830B, a floor 831B, a first water line 895B, a second water line 894B, a pump 816B, a heat exchanger 828B, a pump 817B that pumps water into a distribution device 888B, a first screen 856B, a second screen 893B, sprayers 815B located in a distribution system 814B, biomass 805B, product removal indicated by arrow 858B, a cover 832B, a cone 892B at the bottom of the chamber 850B, a biomass input device 808B, a hole 890B, a gravel layer 804B, a geomembrane 803B, and water 891B. However, a conveyor 865B removes spent solids from the tip of the cone 892B and removes them from a side of the system 800B.

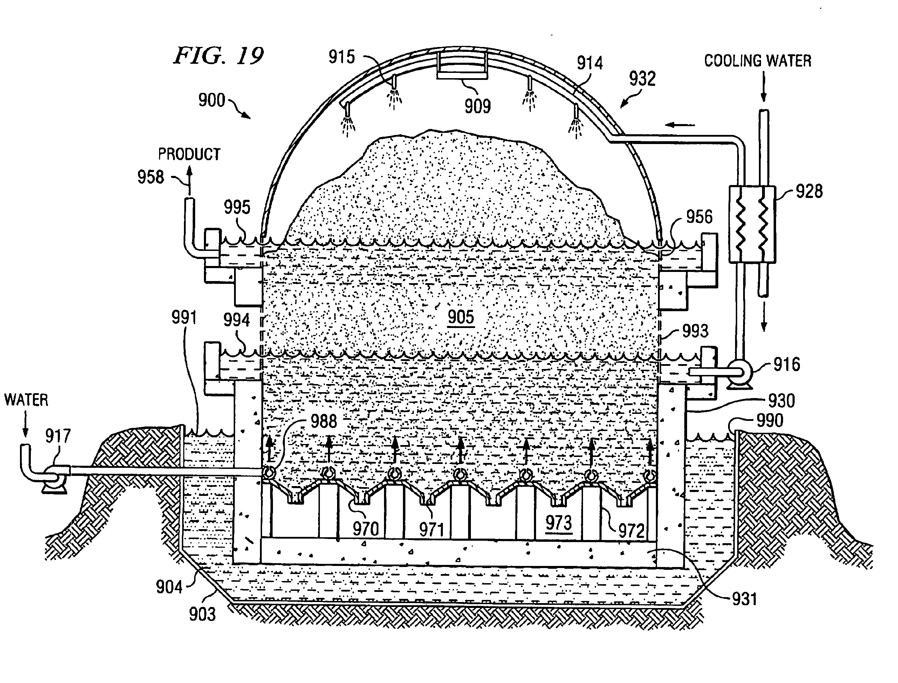

FIGURE 19 shows a system 900 for processing biomass 905, according to another embodiment of the invention. The system 900 of FIGURE 19 is similar to the system 800A of FIGURE 17, including components such as walls 930, a floor 931, a first water line 995, a second water line 994, a pump 916, a heat exchanger 928, a pump 917 that pumps into a distribution device 988, a first screen 956, a second screen 993, sprayers 915 located in a distribution system 914, product removal indicated by arrow 958, a cover 932, a hole 990, a gravel layer 904, a geomembrane 903, and water 991. Biomass 905 may be delivered to the chamber 950 using a conveyor 909 or other suitable device. The system 900 includes multiple cones 970 for removing spent solids. Each of the cones 970 have a passage 971 at the tip and are supported by concrete ribs 972 extending from the floor 931. In particular embodiments, a space 973 created by the concrete ribs 972 may be large enough to allow a person to perform maintenance under the cones 970. In particular embodiments, the space 973 between the ribs 972 may have water circulating so that as solids flow through the cone 970, they are dispersed into a dilute slurry, which is easily pumped. When the dilute slurry is brought to the surface, the dilute slurry may be sent to a settling tank (not shown), where the solids settle out and the liquid is recovered for recycling back to the space 973. **To** help solids flow from the top of the cones 970 to the spaces 973 under the cone, the pressure of liquid circulating in the space 973 may be regulated to be lower than the pressure above the cone 970. This pressure differential forces the solids to flow through the cone 970 when pressure is applied. To help regulate the pressure in the space 973, a gas space may be present under the cones 970, which provides some compressibility.

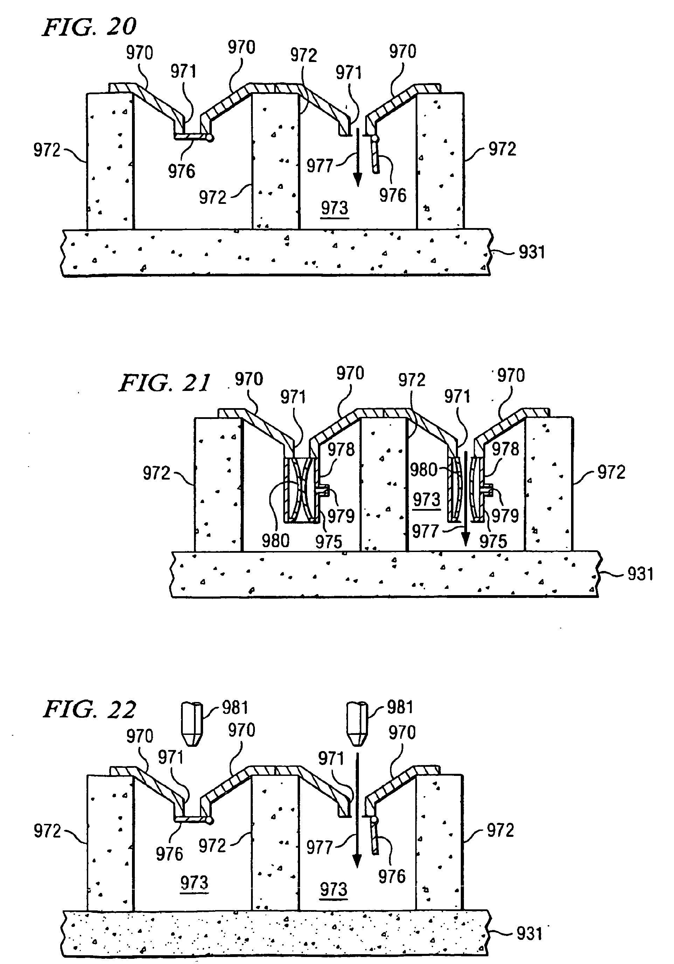

FIGURE 20 shows a cone 970, according to an embodiment of the invention. The cone 970 of FIGURE 20. has a check valve 976 that allows solids to flow in only one direction through the passage 971. In particular embodiments, the check valve 976 may allows solids to be flushed to the space 973 upon application of pressure, for example, a high-pressure liquid or the like. In other embodiments, the check valve 976 may be actuated using a suitable actuator. Upon opening of the check valve 976, solids may flow through the passage 971 in the direction of arrow 977.

FIGURE 21 shows a cone 970, according to an embodiment of the invention. The cone 970 of FIGURE 21 has a pinch valve 978 adjacent a passage 971. The center 980 of the pinch valve 978 is flexible rubber and the exterior is a rigid housing 975. When pressure is applied to the space between the rubber center 980 and the rigid housing 975, for example through opening 979, the rubber center 980 closes. When pressure is removed from the space between the rubber center 980 and the rigid housing 975, the pinch valve 978 opens, allowing the flow through the passage 971 in the direction of arrow 977.

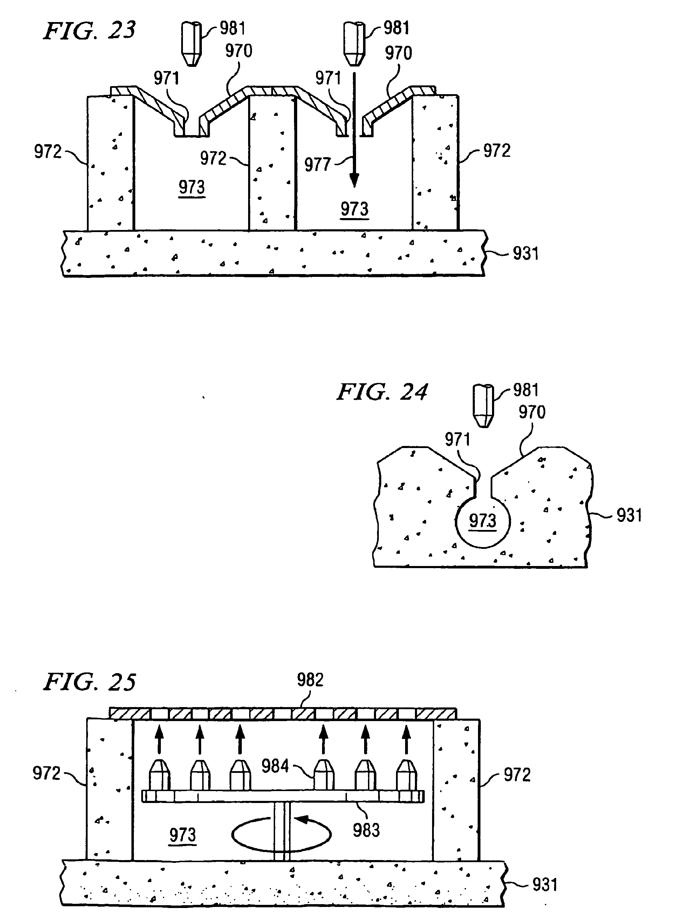

FIGURES 22 and 23 illustrates the use of a jet 981 in conjunction with a cone 970, according to embodiments of the invention. In the embodiments of FIGURE 22 and 23, a jet 981 is added to the space above the cone 970. High pressure fluid (e.g., water) exiting the jet 981 forces solids to flow through the passage 971 of the cone 970, allowing a flow indicated by arrow 977 and preventing blockage. As shown in FIGURE 22, in particular embodiments the jet 981 may be used in conjunction with a check valve 976. As shown in FIGURE 23, in particular embodiments the jet 981 may be used without a check valve 976. In FIGURE 23, the pressure above and below the cone 970 (e.g., on each side of the passage 971) may be substantially the same. Accordingly, high-speed fluid (e.g., water) exiting the nozzle 981 forces solids to flow through the passage 971 to the space 973 below the cone 970, where they can be flushed out in a dilute slurry.

FIGURE 24 shows a cone 970 formed in a floor 931, according to an embodiment of the invention. The cone 970 is formed into the floor 931, which as described above may be made of a variety of materials, including, but not limited to, concrete. In such a embodiment, a space 973 may shaped as a pipe in the floor 931 below the tip of the cone 970 to allow removal solids in a dilute slurry. To enhance flow to the space 973, particular embodiments may use a jet 981 to force solids through a passage 971 at the tip of the cone 970.

FIGURE 25 shows a grate 982, according to embodiment of the invention. The grate 982 in particular embodiments may be placed in similar location to the cones of other embodiments and operate in similar manner. A rotating set 983 of nozzles 984, which may spray in a manner similar to a lawn sprinkler, blasts solids from the grate 982 allowing the solids to drop to the space 973 below where they may be mixed into a dilute circulating slurry, which brings the solids to the surface for ultimate disposal.

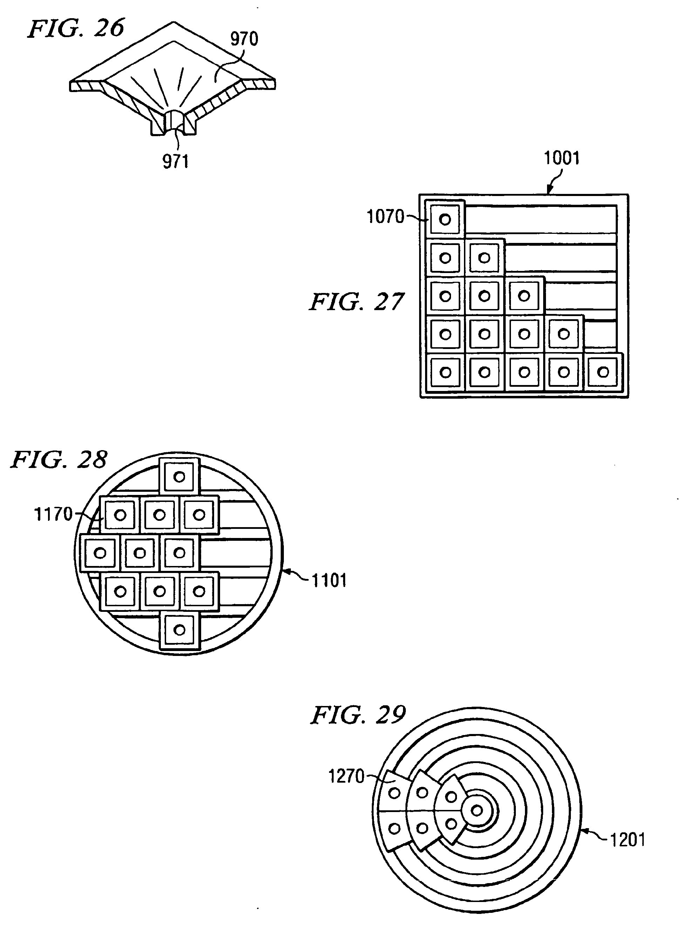

FIGURE 26 shows an isometric view of a cut-away cone 970, according to embodiment of the invention. At the tip of the cone is a passage 971.

FIGURE 27, 28, and 29 show patterns for cones, according to embodiments of the invention. FIGURE 27 shows a pattern 1001 by which cones 1070 may be laid on the bottom of a chamber that has a rectangular base, according to an embodiment of the invention.

FIGURE 28 shows a pattern 1101 by which cones 1170 may be laid on the bottom of a chamber that has a circular base, according to an embodiment of the invention.

FIGURE 29 shows a pattern 1201 by which cones 1270 may be laid on the bottom of a chamber that has a circular base, according to an embodiment of the invention. The pattern 1201 is shown as a series of concentric cones 1270. In other embodiments, the pattern may be a spiral of cones.

Although the present invention has been described with several embodiments, a myriad of changes, variations, alterations, transformations, and modifications may be suggested to one skilled in the art, and it is intended that the present invention encompass such changes, variations, alterations, transformation, and modifications as they fall within the scope of the appended claims.

Further spects of the present invention are set out below in the following numbered paragraphs:
1. A system for processing biomass, the system comprising: a chamber defined by at least a bottom, at least one wall, and a cover supported by the at least one wall; a biomass input device operable to deliver biomass into the chamber to form a biomass pile; a fluid input device operable to deliver fluid into the chamber to the biomass pile; and a retrieval device operable to receive fluid from the chamber.
2. The system of Paragraph 1, wherein the retrieval device is a drainage device, the system further comprising: a pump operable to circulate fluid through the biomass pile by delivering fluid to the fluid input device and receive fluid from the drainage device after the fluid from the fluid input device has traveled through the biomass pile; and an air distribution device operable to deliver air to the biomass pile.
3. The system of Paragraph 2, wherein the cover is a rigid cover.
4. The system of Paragraph 3, wherein the rigid cover is domed.
5. The system of Paragraph 3, wherein the rigid cover is arched.
6. The system of Paragraph 3, wherein the rigid cover comprises an inner core sandwiched between two exterior layers.
7. The system of Paragraph 6, wherein the inner core is made of papercrete and the exterior layers are made of a waterproofing material.
8. The system of Paragraph 7, wherein the inner core comprises at least 50% paper and less than 30% cement.
9. The system of Paragraph 7, wherein the waterproofing material comprises tar.
10. The system of Paragraph 6, wherein the inner core is made of papier mache and the exterior layers are made of a waterproofing material.
11. The system of Paragraph 10, wherein the waterproofing material comprises tar.
12. The system of Paragraph 10, wherein the rigid cover includes a cable operable to hold down the rigid cover.
13. The system of Paragraph 2, wherein the cover comprises a geomembrane supported by a structural member.
14. The system of Paragraph 1, further comprising: a first water line in the chamber, wherein the biomass is at least partially submerged under the first water line.
15. The system of Paragraph 14, wherein a portion of the first water line extends outside of the chamber.
16. The system of Paragraph 15, wherein the retrieval device is operable to retrieve a fluid product from the portion of the first water line that extends outside of the chamber.
17. The system of Paragraph 16, wherein the fluid product is a carboxylate salt.
18. The system of Paragraph 16, further comprising: a screen operable to prevent the biomass from entering the portion of the first water line that extends outside of the chamber.
19. The system of Paragraph 14, wherein the biomass is completely submerged under the first water line, and the fluid input devices deliver fluid into a bottom of the chamber.
20. The system of Paragraph 14, further comprising: a second fluid input device, wherein a portion of the biomass extends above the first water line, the fluid input devices delivers fluid into a bottom of the chamber, and the second fluid input device delivers fluid to the portion of the biomass that extends above the first water line.
21. The system of Paragraph 14, further comprising: a second water line in the chamber, wherein the biomass is at least partially submerged under the second water line.
22. The system of Paragraph 20, wherein: a portion of the biomass extends above the first water line, a portion of the first water line extends outside of the chamber, a portion of the second water line extends outside of the chamber, the retrieval device is operable to retrieve a fluid product from the portion of the first water line that extends outside of the chamber.
23. The system of Paragraph 22, wherein the fluid input devices delivers fluid into a bottom of the chamber, the system further comprising: a second fluid input device, and a pump operable to deliver fluid to the second fluid input device and receive fluid from the portion of the second water line that extends outside of the chamber.
24. The system of Paragraph 23, further comprising: a first screen operable to prevent the biomass from entering the portion of the first water line that extends outside of the chamber, and a second screen operable to prevent the biomass from entering the portion of the second water line that extends outside of the chamber.
25. The system of Paragraph 1, wherein the bottom comprises at least one screw conveyor operable to transport solids out of the chamber.
26. The system of Paragraph 25, wherein the bottom comprises at least one V-shaped section and the at least one screw conveyor is disposed in a tip of the at least one V-shaped section.
27. The system of Paragraph 26, wherein the at least one V-shaped section comprises a plurality of fluid jets operable to spray solids toward the screw conveyor.
28. The system of Paragraph 25, wherein the screw conveyor is a plurality of screw conveyors, and each of the plurality of screw conveyors transports solids to another conveyor, which transports solids out of the chamber.
29. The system of Paragraph 28, wherein the plurality of screw conveyor include at least two sets of screw conveyors, and the other screw conveyor is located between the at least two sets of screw conveyors.
30. The system of Paragraph 25, wherein the bottom includes at least one cone having a tip, and the at least one screw conveyor begins at the tip.
31. The system of Paragraph 30, wherein the at least one screw conveyor transports the solids out through the cover.
32. The system of Paragraph 30, wherein the at least one screw conveyor transports the solids out through a side of the chamber.
33. The system of Paragraph 1, further comprising: a hole operable to surround the bottom, the hole lined with a geomembrane and filled with a gravel layer and water.
34. The system of Paragraph 1, wherein the bottom includes at least one cone having a passage, the passage operable to transport solids to a space below the the bottom.
35. The system of Paragraph 34, wherein the space includes a circulating fluid operable to carry away the solids as a slurry.
36. The system of Paragraph 34, further comprising: a check valve covering the passage, the check valve operable to selectively allow a flow of solids through the passage to the space.
37. The system of Paragraph 34, further comprising: a pinch valve covering the passage, the pinch valve operable to selectively allow a flow of solids through the passage to the space.
38. The system of Paragraph 34, further comprising: a spray nozzle disposed adjacent the passage, the spray nozzle operable to force solids through the passage with a sprayed fluid.
39. The system of Paragraph 38, further comprising: a check valve covering the passage, the check valve operable to selectively allow a flow of solids through the passage to the space.
40. A system for processing biomass, the system comprising: a chamber defined by at least a bottom and an adjustable cover, the adjustable cover having a lowered position and a raised position; a biomass input device operable to deliver biomass into the chamber to form a biomass pile; and a fluid input device operable to deliver fluid into the chamber to the biomass pile.
41. The system of Paragraph 40, further comprising: at least one pole operable to support the adjustable cover.
42. The system of Paragraph 41, further comprising: a winch system disposed on top of the at least one pole, the winch system operable to elevate the adjustable cover from the lowered position to the raised position.
43. The system of Paragraph 42, further comprising: a bellows surrounding the winch system.
44. The system of Paragraph 41, wherein the pole includes perforations, and the pole serves as both the biomass input device and the fluid input device by delivering fluid and biomass through the perforations.
45. A system for processing biomass, the system comprising: a chamber defined by at least a bottom and an rigid cover; a biomass input device operable to deliver biomass into the chamber to form a biomass pile; a fluid input device operable to deliver fluid into the chamber to the biomass pile; a pump operable to circulate fluid through the biomass pile by delivering fluid to the fluid input device and receive fluid from a drainage device after the fluid from the fluid input device has traveled through the biomass pile; and an air distribution device operable to deliver air to the biomass pile.
46. The system of Paragraph 45, wherein the rigid cover is spherical.
47. The system of Paragraph 45, wherein the rigid cover is arched.
48. The system of Paragraph 45, wherein the rigid cover comprises an inner core sandwiched between two exterior layers.
49. The system of Paragraph 48, wherein the inner core is made of papercrete and the exterior layers are made of a waterproofing material.
50. The system of Paragraph 49, wherein the inner core comprises at least 50% paper and less than 30% cement.
51. The system of Paragraph 49, wherein the waterproofing material comprises tar.
52. The system of Paragraph 48, wherein the inner core is made of papier mache and the exterior layers are made of a waterproofing material.
53. The system of Paragraph 52, wherein the waterproofing material comprises tar.
54. The system of Paragraph 45, wherein the rigid cover includes a cable operable to hold down the rigid cover.
55. A system for processing biomass, the system comprising: a chamber defined by at least a bottom, at least one wall, and a cover supported by the at least one wall; a biomass input device operable to deliver biomass into the chamber to form a biomass pile; a fluid input device operable to deliver fluid into the chamber; a first water line at least partially submerging the biomass, a portion of the first water line extending outside of the chamber; a retrieval device operable to retrieve a fluid product from the portion of the first water line that extends outside of the chamber; and a screen operable to prevent the biomass from entering the portion of the water line that extends outside of the chamber.
56. The system of Paragraph 55, further comprising: a second fluid input device, wherein a portion of the biomass extends above the first water line, the fluid input devices delivers fluid into a bottom of the chamber, and the second fluid input device delivers fluid to the portion of the biomass that extends above the first water line.
57. The system of Paragraph 55, wherein the bottom comprises at least one screw conveyor operable to transport solids out of the chamber.
58. The system of Paragraph 57, wherein the bottom comprises at least one V-shaped section and the at least one screw conveyor is disposed in a tip of the at least one V-shaped section.
59. The system of Paragraph 58, wherein the at least V- shaped section comprises a plurality of fluid jets operable to spray solids toward the screw conveyor.
60. The system of Paragraph 57, wherein the screw conveyor is a plurality of screw conveyors, and each of the plurality of screw conveyors transports solids to another conveyor, which transports solids out of the chamber.
61. The system of Paragraph 60, wherein the plurality of screw conveyors include at least two sets of screw conveyors, and the other screw is located between the at least two sets of screw conveyors.
62. The system of Paragraph 57, wherein the bottom includes at least one cone having a tip, and the at least one screw conveyor begins at the tip.
63. The system of Paragraph 62, wherein the at least one screw conveyor transports the solids out through the cover.
64. The system of Paragraph 62, wherein the at least one screw conveyor transports the solids out through a side of the chamber.
65. The system of Paragraph 55, further comprising: a hole operable to surround the bottom, the hole lined with a geomembrane and filled with a gravel layer and water.
66. The system of Paragraph 55, wherein the bottom includes at least one cone having a passage, the passage operable to transport solids to a space under the bottom.
67. The system of Paragraph 66, wherein the space includes a circulating fluid operable to carry away the solids as a slurry.
68. The system of Paragraph 67, further comprising: a check valve covering the passage, the check valve operable to selectively allow a flow of solids through the passage to the space.
69. The system of Paragraph 67, further comprising: a pinch valve covering the passage, the pinch valve operable to selectively allow a flow of solids through the passage to the space.
70. The system of Paragraph 67, further comprising: a spray nozzle disposed adjacent the passage, the spray nozzle operable to force solids through the passage with a sprayed fluid.
71. The system of Paragraph 70, further comprising: a check valve covering the passage, the check valve operable to selectively allow a flow of solids through the passage to the space.
72. A method of processing biomass, the method comprising: transporting biomass to a chamber to form a biomass pile, the chamber defined by at least a bottom and adjustable cover; transferring fluids to the chamber; lowering the adjustable cover from a raised position to a lowered position; processing the biomass with the adjustable cover in the lowered position.
73. The method of Paragraph 72, wherein processing the biomass includes fermenting the biomass.
74. The method of Paragraph 72, wherein the adjustable cover in the lowered position is adjacent the biomass pile.
75. The method of Paragraph 72, further comprising: supporting the adjustable cover with a pole that has perforations, wherein transporting the biomass to the chamber to form the biomass pile and transferring fluids to the chamber are carried out through the perforations.
76. The method of Paragraph 72, wherein lowering the adjustable cover from a raised position to a lowered position is carried out by a winch located on the pole, the winch attached to the adjustable cover via a cable and releasing the cable to lower the adjustable cover.
77. A system for processing biomass, the system comprising: a plurality of chambers, each of the plurality of chambers defined by at least a bottom and a cover; at least one biomass input device operable to deliver biomass into the plurality of chambers; at least one fluid input device operable to deliver fluid into the plurality of chambers; at least one fluid retrieval device operable to receive fluid from the plurality of chambers; and wherein each of the plurality of chambers are operable to have different stages of biomass processing.
78. The system of Paragraph 77, further wherein at least two of the plurality of chambers are further defined by a common wall.
79. The system of Paragraph 78, wherein at least some of the plurality of chambers have flexible covers.
80. The system of Paragraph 78, wherein at least some of the plurality of chambers have rigid covers.
81. The system of Paragraph 77, further comprising: at least one solids retrieval device operable to retrieve solids from the plurality of chambers.

## Claims

1. A process comprising
adding biomass into a vessel;
adding an inoculum into said vessel;
introducing liquid into said vessel from the bottom of the vessel to form a liquid level, wherein said liquid submerges at least a portion of the biomass;
fermenting the biomass in said vessel to produce a carboxylate salt; and removing spent biomass from the bottom of the vessel.

2. The process of claim 1 further comprising introducing calcium carbonate into said vessel prior to fermenting said biomass.

3. The process of claim 1 further comprising
withdrawing a portion of the liquid in the vessel; and
spraying the biomass above the liquid level with said portion of liquid, said spraying optionally being performed intermittently.

4. The process of claim 3 further comprising regulating the temperature of the portion of liquid withdrawn from the vessel, said temperature regulation optionally comprising passing said portion of liquid through a heat exchanger.

5. The process of claim 1 further comprising controlling the temperature of the biomass by regulating the temperature of the liquid in said vessel.

6. The process of claim 1 further comprising extracting the liquid from said vessel to recover said carboxylate salt, said liquid extraction optionally comprising withdrawing the liquid at a position close to the liquid level.

7. The process of claim 1 wherein adding the biomass and removing the biomass are performed continuously.

8. The process of claim 1 further comprising adding the removed biomass from said vessel into a second vessel, wherein the process of claim 1 takes place in said second vessel.

9. A method comprising
forming a first pile comprising biomass;
inoculating said first pile comprising biomass;
circulating a liquid in said first pile ;
fermenting the biomass in said first pile to produce a carboxylate salt; and extracting at least a portion of the liquid in the first pile and passing said portion of liquid to a second pile comprising biomass, wherein said second pile is operating at a higher carboxylate salt concentration than said first pile.

10. The method of claim 9 further comprising introducing calcium carbonate into the first pile while said first pile comprising biomass is being formed.

11. The method of claim 9 wherein circulating liquid in said first pile is performed intermittently.

12. The method of claim 9 further comprising pretreating the biomass prior to inoculating said first pile comprising biomass, wherein pretreating and fermenting said biomass take place in the same enclosure without transferring the biomass, said biomass pretreatment optionally comprising introducing into the biomass lime, or lime and air, said air optionally being scrubbed of carbon dioxide prior to being introduced into the biomass.

13. The method of claim 9 further comprising handling a multiplicity of piles comprising biomass in a round-robin manner.

14. The method of claim 9 further comprising regulating the temperature of the circulating liquid in said first pile, said temperature regulation optionally comprising passing said circulating liquid through a heat exchanger.

15. The method of claim 9 further comprising controlling the temperature of the biomass in said first pile by regulating the temperature of the circulating liquid in said first pile.
